## Europäisches Patentamt
## European Patent Office
## Office européen des brevets

(11) Veröffentlichungsnummer: **0 418 827 B1**

(12) **EUROPÄISCHE PATENTSCHRIFT**

(45) Veröffentlichungstag der Patentschrift: **20.12.95**

(21) Anmeldenummer: **90117943.2**

(22) Anmeldetag: **18.09.90**

Teilanmeldung 94112145.1 eingereicht am 18/09/90.

(51) Int. Cl.⁶: **A61K 39/40**, C12P 21/08, A61K 39/02, C07K 14/005, C12N 15/63, C12N 1/20

(54) **Impfstoff gegen die Lyme-Krankheit**

(30) Priorität: **19.09.89 DE 3931236**
**17.05.90 DE 4015911**

(43) Veröffentlichungstag der Anmeldung:
**27.03.91 Patentblatt 91/13**

(45) Bekanntmachung des Hinweises auf die Patenterteilung:
**20.12.95 Patentblatt 95/51**

(84) Benannte Vertragsstaaten:
**AT BE CH DE DK ES FR GB GR IT LI LU NL SE**

(56) Entgegenhaltungen:
**EP-A- 0 252 641**

**PROC. NATL. ACAD. SCI., US, Band 87, Mai 1990, Seiten 3768-3772; U.E. SCHAIBLE et al.: "Monoclonal antibodies specific for the outer surface protein A (OspA) of Borrelia burgdorferi prevent Lyme borreliosis in severe combined immunodeficiency (scid) mice"**

(73) Patentinhaber: **Max-Planck-Gesellschaft zur Förderung der Wissenschaften e.V.**
**Bunsenstrasse 10**
**D-37073 Göttingen (DE)**

Patentinhaber: **Deutsches Krebsforschungszentrum Stiftung des öffentlichen Rechts**
**Im Neuenheimer Feld 280**
**D-69120 Heidelberg (DE)**

(72) Erfinder: **Simon, Markus M., Dr.**
**Kartäuserstr. 35**
**D-7800 Freiburg (DE)**
Erfinder: **Schaible, Ulrich E.**
**Zähringerstr. 2**
**D-7800 Freiburg (DE)**
Erfinder: **Eichmann, Klaus, Prof.Dr.**
**Reutebachgasse 38f**
**D-7800 Freiburg (DE)**
Erfinder: **Kramer, Michael, Dr.**
**Im Neuenheimer Feld 324**
**D-6900 Heidelberg (DE)**
Erfinder: **Reinhard, Wallich, Dr.**
**Unteren Rombach 6**
**D-6900 Heidelberg (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B.**
**Böhm**
**Postfach 86 08 20**
**D-81635 München (DE)**

**Beschreibung**

Die Lyme-Borreliose ist die häufigste, von Zecken übertragene Infektionskrankheit in den gemäßigten Breiten. Sie wird durch die Spirochäte Borrelia burgdorferi hervorgerufen, die vor allem durch Zecken des Stamms Ixodes auf den Menschen übertragen wird. Die Krankheit ist eine chronische progressive Infektion, die viele Organe, wie die Haut, das zentrale und periphere Nervensystem, das Herz, die Leber, die Niere und das musculoskeletale System befällt. Da eine zuverlässige Behandlung dieser Krankheit durch Therapie mit Antibiotika schwierig ist, werden gegenwärtig große Anstrengungen unternommen, den Erreger selbst und die Immunantwort des Wirts auf Infektion mit B. burgdorferi zu erforschen. Bei von der Lyme-Krankheit betroffenen Personen wird zwar ein hoher Titer an Antikörpern gegen B. burgdorferi festgestellt, der aber keinen Schutz gegen die Infektion bewirkt. Es wird angenommen, daß der Erreger sehr rasch aus der Blutbahn in das Gewebe übertritt und dort für das Immunsystem nicht mehr unmittelbar erreichbar ist. Dies würde bedeuten, daß ein Schutz durch Antikörper nur unmittelbar nach Beginn der Infektion, solange also die Erreger sich noch in der Blutbahn befinden, möglich ist.

Die Tatsache, daß eine natürliche Infektion mit B. burgdorferi in verschiedenen Tierarten gefunden wurde, hat zum Versuch geführt, Labormodelle für die Lyme-Krankheit zu etablieren. Dies gelang auch mit begrenztem Erfolg. So wurde bei Experimenten, welche die Induzierung einer für B. burgdorferi spezifischen Immunantwort in der Maus zum Ziel hatten, gefunden, daß die Infektion von Inzucht-Mausstämmen mit einem schon lange Zeit kultivierten B. burgdorferi-Isolat zu mäßigen, aber signifikanten pathomorphologischen Veränderungen in verschiedenen Organen wie dem Gehirn, dem Herz, den Lungen und den Nieren führte, die vergleichbar mit denjenigen waren, die bei Patienten mit Lyme-Krankheit zu beobachten sind (Schaible et al., (1988) Infect. Immun. 1, 41). Die Ausbildung eines ernsteren Krankheitsbildes bei Tieren wurde vermutlich entweder durch die Immunabwehr des Wirts und/oder durch die reduzierte Virulenz von für einen längeren Zeitraum in vitro kultivierten Spirochäten (Johnson et al., (1984), J. Clin. Microbiol. 20, 747; Schwan et al., (1988), Infect. and Immun. 56, 1837) verhindert.

Howe et al (Infection and Immunity 54 (1986), 207-212) offenbaren, daß die für die Außenmembranproteine OspA und OspB von Borrelia burgdorferi kodierenden Gene innerhalb einer einzigen Transkriptionseinheit lokalisiert sind. Bergström et al (Mol. Microbiol. 3 (1989), 479-486) beschreiben eine molekulare Analyse der für die Proteine OspA und OspB aus den B. burgdorferi Stamm B31 kodierenden DNA-Sequenzen. Wilske et al (Ann. N. Y. Acad. 539 (1988), 126-143) beschreiben eine Variabilität der Oberflächenantigene in verschiedenen B. burgdorferi Stämmen.

Die der Erfindung zugrundeliegende Aufgabe ist es, einen wirksamen Impfstoff gegen die Lyme-Krankheit bereitzustellen. Dazu ist jedoch zunächst die Entwicklung eines geeigneten tierischen Labormodells erforderlich. Es wird nun vorgeschlagen, daß ein Maus-Stamm ohne funktionsfähige T- und B-Zellen, die sogenannte Scid-Maus (Bosma et al., (1983) Nature 10, 52), als Versuchstier dienen kann, da Scid-Mäuse bei Infektion mit einem pathogenen B. burgdorferi-Isolat eine multisystemische Krankheit und zwar hauptsächlich Polyarthrithis und Carditis, entwickeln. Durch dieses Tiermodell wird es erst möglich, die Wirkung von Impfstoffen gegen die Lyme-Krankheit zu erproben.

Ein Gegenstand der Erfindung ist ein passiver Impfstoff gegen die Lyme-Krankheit, der einen oder mehrere für das 31kD Antigen (OspA) oder/und das 34 kD Antigen (OspB) von B. burgdorferi, insbesondere OspA oder/und OspB von B. burgdorferi der Stämme B31 (ATCC 35210) oder/und ZS7 (DSM 5527) spezifische monoklonale Antikörper enthält. Bevorzugt ist ein Impfstoff, der einen erfindungsgemäßen Antikörper der Klasse IgG, besonders bevorzugt der Subklasse IgG2b oder IgG1, enthält. Die Verabreichung des erfindungsgemäßen Antikörpers bewirkt überraschenderweise im Gegensatz zur Verabreichung eines anderen Antikörpers, z.B. gegen das 41 kD Oberflächenantigen von B. burgdorferi (Flagellin) bei immundefizienten Versuchstieren, vorzugsweise Scid-Mäusen, die mit lebensfähigen pathogenen B. burgdorferi, vorzugsweise B. burgdorferi ZS7 infiziert wurden, daß die Ausbildung von Arthritis, Carditis und Hepatitis vollständig oder zumindest weitgehend verhindert wird.

Gegebenenfalls kann der erfindungsgemäße Impfstoff mit dem Antikörper als Wirkstoff auch noch übliche Träger-, Füll- und Hilfsstoffe enthalten.

Weiterhin beinhaltet die Erfindung auch ein Verfahren zur Gewinnung eines passiven Impfstoffs gegen die Lyme-Krankheit aus Lymphozyten oder Milzzellen eines Versuchstieres, vorzugsweise einer Maus, die mit B. burgdorferi Organismen oder Teilen davon, vorzugsweise mit kompletten B. burgdorferi B31 oder/und ZS7 Organismen, immunisiert ist, wobei man aus dem Lymphozyten oder Milzzellen des immunisierten Tieres durch Zellfusion ein Hybridom gewinnt, das einen erfindungsgemäßen monoklonalen Antikörper produziert.

Eine Hybridoma-Zellinie (ECACC 89 09 1302), die einen erfindungsgemäßen Antikörper LA-2 gegen OspA (IgG2b) produziert ist auch beschrieben. Weiterhin sind auch die den Antikörper LA-26.1 gegen OspA (IgG1) produzierende Hybridoma-Zellinie ECACC 90050406, sowie die Antikörper LA-25.1 bzw. LA-27.1 gegen OspB (IgG2b bzw. IgG1) produzierenden Hybridoma-Zellinien ECACC 90050405 bzw. ECACC 90050407 beschrieben.

Ebenso ist der pathogene B. burgdorferi Stamm ZS7 (DSM 5527) angegeben.

Weiterhin ist ein Antigen beschrieben, das mit einem erfindungsgemäßen monoklonalen Antikörper immunreagiert. Darunter ist ein Antigen zu verstehen, welches die gesamte Aminosäuresequenz von OspA bzw. OspB oder auch nur eine immunogen wirkende Teilsequenz (immunogenes Epitop) von OspA bzw. OspB enthält. Potentiell immunogene Epitope dieser Proteine kann ein Fachmann ohne Schwierigkeiten durch eine Strukturanalyse des OspA-Protein (z.B. Chou-Fassmann Analyse) ermitteln und dann experimentell auf ihre Wirksamkeit testen.

Insbesondere ist ein rekombinantes Antigen beschrieben, das mit dem erfindungsgemäßen Antikörper immunreagiert, wobei sich die für das Antigen kodierende DNA-Sequenz auf einem rekombinanten Vektor, vorzugsweise einem prokaryontischen Vektor befindet, der geeignet zur Proteinexpression ist.

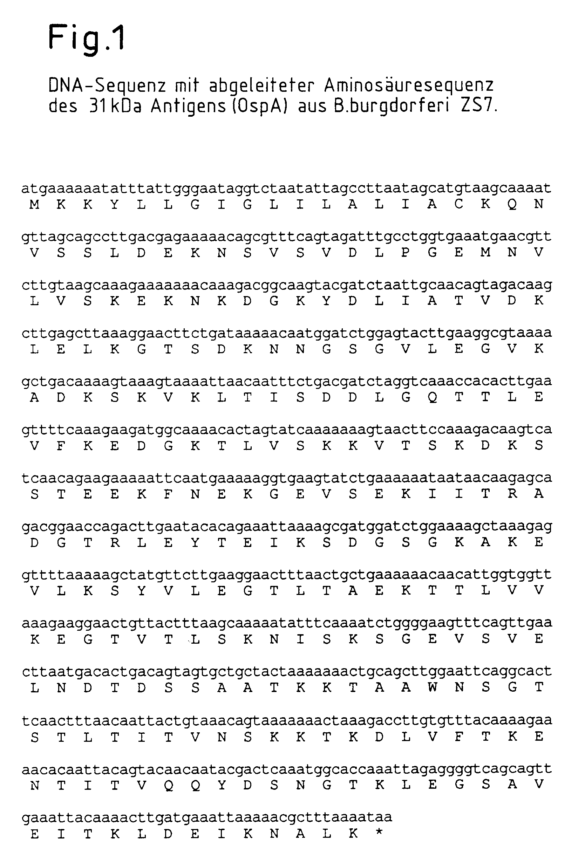

Insbesondere ist ein Antigen aus B. burgdorferi ZS7 beschrieben, das spezifisch mit dem erfindungsgemäßen Antikörper immunreagiert und die in Abb. 1 gezeigte Aminosäuresequenz oder ein immunogenes Epitop aus dieser Sequenz enthält. Demgemäß ist auch eine rekombinante DNA beschrieben, welche (1) die in Abb. 1 gezeigte, (2) eine, ihr im Rahmen der Degeneration des genetischen Codes entsprechende Nukleinsäuresequenz oder (3) eine mit einer Sequenz aus (1) oder/und (2) unter stringenten Bedingungen hybridisierende Sequenz enthält, die für das 31 kD Antigen von B. burgdorferi ZS7 oder ein immunogenes Epitop davon kodiert. Der Begriff "stringente Hybridisierungsbedingungen" ist dabei wie in Maniatis et al., Molecular Cloning. A Laboratory Manual (1982), Cold Spring Harbor Laboratory, New York, zu verstehen.

Besonders bevorzugt ist ein Antigen, das ein rekombinantes Nicht-Fusionsprotein oder $\beta$-Galactosidase-Fusionsprotein ist.

Weiterhin ist einen rekombinanten Vektor, der eine oder mehrere Kopien einer erfindungsgemäßen rekombinanten DNA enthält beschrieben. Der erfindungsgemäße Vektor kann ein prokaryontischer oder/und eukaryontischer Vektor sein, er ist vorzugsweise ein prokaryontischer Vektor. Der rekombinante Vektor kann in der Wirtszelle extrachromosomal vorliegen (z.B. Plasmid) oder er kann sich auch in das Genom der Wirtszelle integrieren (z.B. Bakteriophage Lambda). Vorzugsweise ist der erfindungsgemäße Vektor ein Plasmid. Besonders bevorzugt ist der rekombinante Vektor PZS-7/31-2 (DSM 5528).

Des weiteren ein Verfahren zur Gewinnung von erfindungsgemäßen Antigenen durch Untersuchung einer B. burgdorferi Genbank mit einem oder mehreren erfindungsgemäßen Antikörpern angegeben, wobei man die Klone isoliert, welche mit den verwendeten Antikörpern eine positive Immunreaktion zeigen.

Weiterhin ist ein Verfahren zur Gewinnung eines passiven Impfstoffs gegen die Lyme-Krankheit beschrieben, wobei man Versuchstiere, vorzugsweise Mäuse, mit dem Antigen immunisiert und aus dem immunisierten Versuchstier auf übliche Weise protektive, monoklonale Antikörper gewinnt.

Schließlich ist noch ein Verfahren beschrieben zur Isolierung und Rekultivierung von pathogenen B. burgdorferi Organismen, die dadurch gekennzeichnet sind, daß man aus immundefizienten Versuchstieren, vorzugsweise Mäusen, die zuvor mit dem Erreger infiziert werden, den Erreger gewinnt, wobei die Pathogenität des Erregers erhalten bleibt. Besonders bevorzugt ist ein Verfahren, bei dem man pathogene B. burgdorferi ZS7 (DSM 5527) Organismen aus Blut oder/und Gelenken von infizierten Scid-Mäusen gewinnt.

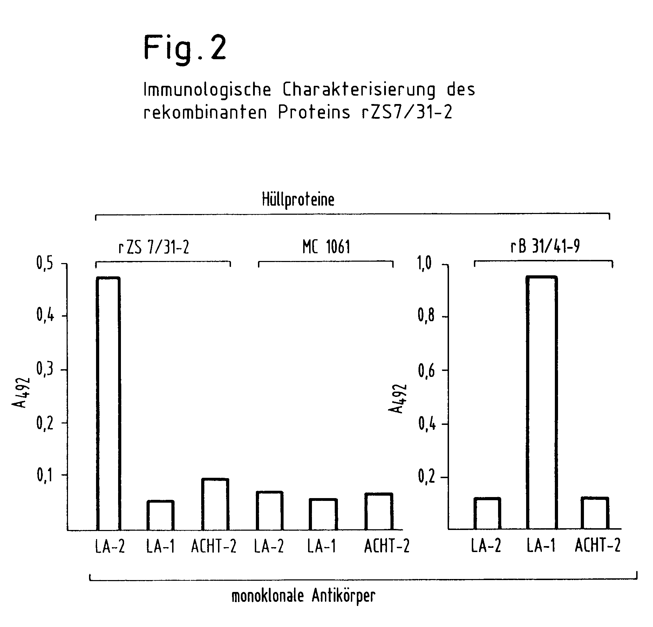

Die Verdeutlichung der Erfindung erfolgt durch nachfolgende Beispiele und die Abbildungen 1 und 2.

Es zeigen:

Abb. 1 die DNA- und Aminosäuresequenz des 31 kD Antigens (OspA) aus B. burgdorferi ZS7,

Abb. 2 die immunologische Charakterisierung des rekombinanten Proteins rZS7/31-2.

## Beispiel 1

Induzierung von Arthritis, Carditis und Hepatitis in Scid-Mäusen durch Infektion mit B. burgdorferi Stamm ZS7.

Behandlung der Mäuse mit B. burgdorferi

Erwachsenen Mäusen der Stämme C.B-17 Scid (homozygot für die Scid-Mutation) und C.B-17 wurden $1\times10^5$, $5\times10^5$, $1\times10^6$ oder $1\times10^8$ lebensfähige oder abgetötete (UV-Strahlung) B. burgdorferi Organismen subkutan in die Schwanzwurzel injiziert.

Isolierung von B. burgdorferi aus Zecken und Mäusen

Die Untersuchungen wurden mit dem schon seit langem kultivierten B. burgdorferi Stamm B31 (ATCC 35210) und dem frischen Isolat B. burgdorferi ZS7 (DSM 5527), das aus einer weiblichen Ixodes rizinus Zecke isoliert wurde, durchgeführt. Alle B. burgdorferi Stämme wurden in modifiziertem Kelly's Medium kultiviert (Barbour et al., (1983) Switzerland. Curr. Microbiol. 8, 123). B. burgdorferi Organismen, die aus dem Mitteldarm von mit Ethanol sterilisierten Zecken oder aus Blut von infizierten Mäusen gewonnen wurden, wurden anfangs in Kelly's Medium unter Zusatz von 8 $\mu$g/ml Kanamycin und 230 $\mu$g/ml Fluoruracil kultiviert (Johnson et al., (1984) J. Clin. Microbiol. 1, 81).

Serologische Tests

Die Detektion von B. burgdorferi spezifischen Antikörpern wurde in einem konventionellen ELISA-Verfahren durchgeführt (Justus et al., (1988) Wehrmed. Mschr. 32, 263). Die Standardkurve für den Gehalt an Immunglobulin (Ig) wurde durch Beschichtung einer Schale mit Anti-Maus Ig (1:500 Verdünnung der Serumlösung von Paesel, Frankfurt, BRD) und Titration des Gesamt-Maus IgG-oder IgM-Gehalts (Calbiochem., LaJolla, USA) erhalten. Gesamtserum IgM und IgG wurde ähnlich gemessen. Die Konzentration von B. burgdorferi spezifischen IgM- oder IgG-Antikörpern ist in $\mu$g Ig/ml Serum angegeben.

Immunfluoreszenz und Giemsa-Anfärbung

50 $\mu$l Blut wurden in einen Hematocrit-Röhrchen pipettiert (Becton und Dickinson, Heidelberg, BRD) und bei 5.000 g in einer Hematocrit-Zentrifuge (ECCO, BRD) zentrifugiert. Die Röhrchen wurden an der Interphase zwischen Serum und Erythrozyten aufgeschnitten und 5 $\mu$l des Serums wurden auf Objektträger aufgetragen (Superior, Bad Mergentheim, BRD). Die mit den Serumproben beladenen Objektträger wurden an der Luft getrocknet und in 100 % Ethanol eine Minute lang bei -20°C fixiert. Nach einstündiger Inkubation mit Kaninchen-Anti B. burgdorferi Hyperimmunserum (1:100 Verdünnung) bei Raumtemperatur wurden die Objektträger fünfmal in PBS gewaschen und dann mit FITC konjugiertem Ziegen-Antikaninchen-Antiserum (1:20 Verdünnung, Jackson Lab., West Grove, USA) eine Stunde lang angefärbt. Die Objektträger wurden gewaschen und in Kaiser's Glycerin-Gelatine (Merck, Darmstadt, BRD) eingebettet und sofort fluoreszenzmikroskopisch untersucht. Unbehandelte Bluttropfen wurden an der Luft getrocknet, in Methanol fixiert, mit Giemsa (0,1 % Merck, Darmstadt, BRD) angefärbt, in PBS entfärbt und in Entellan (Merck, Darmstadt, BRD) eingebettet.

Histologische Präparationen und Färbeverfahren

Verschiedene innere Organe (Gehirn, Herz, Lunge, Leber, Nieren, Milz und Gelenke) wurden von zuvor mit B. burgdorferi infizierten Mäusen zu unterschiedlichen Zeitpunkten nach der Infektion entfernt und entweder in flüssigem Stickstoff zur Präparation von Gefrierschnitten oder in 5 % Formaldehyd (in PBS) zur Einbettung in Paraffin oder Methacrylat aufbewahrt. Schnitte von 4 bis 7 $\mu$m wurden hergestellt, mit Hematoxylin-Eosin angefärbt und in Entellan (Merck AG, Darmstadt, BRD) eingebettet. Die Immunhistologie wurde unter Verwendung des Streptavidin-Biotin-Peroxidase-Systems durchgeführt (Kramer et al., (1989) Eur. J. Immunol. 19, 151).

Tabelle 1 zeigt, daß B. burgdorferi Organismen der Isolate ZS7 und B31 während der gesamten Versuchsdauer im Blut von Scid-Mäusen nachgewiesen wurden, die zuvor mit lebensfähigen Organismen geimpft wurden. Allerdings konnten nur Spirochäten vom Stamm ZS7, aber keine vom Stamm B31 in vitro rekultiviert werden. Bei Vergleich der rekultivierten Organismen mit dem primären B. burgdorferi ZS7 Isolat konnten keine Veränderungen im Proteingehalt oder im Plasmidprofil festgestellt werden. Keine oder nur äußerst geringe Titer an irrelevanten Antikörpern wurden in mit B. burgdorferi infizierten Scid-Mäusen während der gesamten Beobachtungsdauer nachgewiesen. In diesen Tieren wurden keine für B. burgdorferi spezifische IgM- oder IgG-Antikörper gefunden (Tabelle 1). Dagegen exprimierten alle C.B-17-Kontrollmäuse, die mit B. burgdorferi infiziert wurden, große Mengen an Gesamt-Ig und erhöhte Titer an für B. burgdorferi spezifischen IgM- und IgG-Antikörpern. Zwischen 7 und 20 Tagen nach der Infektion mit B. burgdorferi ZS7 zeigten die Scid-Mäuse erste klinische Symptome von Arthritis (Rötung und Schwellung beider Tibiotarsalgelenke), die mit der Zeit zunahmen. Dagegen wurden keine Symptome von Arthritis in Scid-Mäusen, die entweder mit UV-bestrahlten B. burgdorferi ZS7 oder mit lebensfähigen B. burgdorferi B31 Organismen infiziert wurden, und in C.B-17-Kontrollmäusen, die mit lebensfähigen B. burgdorferi ZS7 Organismen infiziert wurden, gefunden.

Auch histopathologisch wurden arthritische Gelenksveränderungen in Scid-Mäusen nachgewiesen, die mit lebensfähigen B. burgdorferi ZS7 infiziert wurden (Tabelle 1). Es wurden schwere Gelenkschädigungen festgestellt, gekennzeichnet durch die Anwesenheit von hyperplastisch entzündeten Synovial-Auskleidungszellen, verbunden mit Erosion und Zerstörung von Knorpelgewebe und/oder Knochen. Weiterhin wurde Pancarditis mit Infiltration von Mononuklearzellen in das Endocardium, Myocardium und Pericardium festgestellt. Ebenfalls wurde eine progressive Entzündung der Leber festgestellt, wobei eine Infiltration von Mononuklearzellen, die auf den Pfortaderbereich und die Zentralvene beschränkt war, granulomatöse Reaktionen und schließlich das Auftreten von Leberfibrose beobachtet wurde. Zusätzlich wurden geringere Schäden in den Nieren, der Lunge, dem Gehirn und der gestreiften Muskulatur festgestellt.

T A B E L L E    1

Infektion von C.B-17 Scid-Mäusen und C.B-17 Kontroll-Mäusen mit B. burgdorferi;
Reisolierung von Spirochäten aus dem Gewebe; Antikörpertiter und Ausbildung von Arthritis

| Mäuse-Stamm | B. burgdorferi Stamm | Anzahl bei Infektion | Tage nach Infektion | B. burgdorferi Detektion im Blut* | Isolierung** | Arthritis klinisch° | Histopathologisch | Antikörper (µg/ml)°° Gesamt Ig µ | Gesamt Ig γ | spezifische Ig µ | spezifische Ig γ |
|---|---|---|---|---|---|---|---|---|---|---|---|
| C.B-17 scid (n=1) | ZS7 | $5 \times 10^5$ | 7 | + | . | . | . | . | 20 | . | . |
| | | | 36 | + | +B | + | + | − | 21 | − | − |
| | | | 49 | + | +B | + | + | − | 26 | − | − |
| | | | 59 | + | +B | + | + | − | 396 | − | − |
| | ZS7 | $1 \times 10^8$ | 7 | + | . | + | + | . | 108 | . | . |
| | | | 23 | + | +B | + | + | − | 54 | − | − |
| | ZS7 | $1 \times 10^8$ | 22 | + | . | + | + | . | 41 | . | . |
| | | | 29 | + | +G | + | + | − | − | − | − |
| | | | 87 | + | +B/G | + | + | − | − | − | − |
| | ZS7 | $1 \times 10^5$ | - | n.b. | n.b. | + | n.b. | n.b. | | n.b. | |
| | | $1 \times 10^6$ | - | n.b. | n.b. | + | n.b. | n.b. | | n.b. | |
| | | $1 \times 10^8$ | 16 | + | + | + | + | . | . | . | . |
| | ZS7uv$_{tr}$ | $1 \times 10^8$ | 16 | - | . | . | . | . | . | . | . |
| | B31 | $1 \times 10^8$ | 22 | + | . | . | . | 26 | 37 | . | . |
| | | | 29 | + | − | − | − | − | − | − | − |
| | − | | 22 | − | − | − | − | − | 47 | − | − |
| | − | | 29 | − | − | − | − | 54 | 364 | − | − |
| C.B-17 (n=7) | ZS7 | $1 \times 10^8$ | 16 | - | . | . | . | 2.515 | 5.963 | 438 | 56 |
| | | | 24 | − | − | − | − | 2.145 | 6.374 | 506 | 94 |
| | − | − | − | − | − | − | − | 304 | 3.804 | − | − |
| | − | − | − | − | − | − | − | 216 | 1.952 | − | − |

*: Durch Giemsa-Färbung oder Immunfluoreszenz; **: Isolierung aus Blut (B), Gelenken (G);
°+: Rötung und Schwellung der tibiotarsalen Gelenke.
°°−: < 7,5 µg/ml Serum

EP 0 418 827 B1

**Beispiel 2**

Wirkung eines für das B. burgdorferi 31kD Antigen spezifischen monoklonalen Antikörpers auf den Verlauf der Lyme-Borreliose in Scid-Mäusen

Herstellung des monoklonalen Antikörpers

Bei Immunisierung einer Maus, die ein intaktes Immunsystem besitzt, mit B. burgdorferi Organismen werden polyklonale Antikörper exprimiert, die für B. burgdorferi spezifisch sind (siehe Tabelle 1).

Zehn Wochen alte weibliche Mäuse des Inzuchtstammes BALB/c wurden mit durch Beschallung homogenisierten Borrelien (B. burgdorferi, Stamm B31; ATCC 35 210) immunisiert.

Immunisierungsprotokoll:

Tag 0:                 200 $\mu$g Borrelien-Antigen in kompletten Freund's Adjuvants subkutan
Tag 21, 35, 49, 63:    Challenge mit 100 $\mu$g Borrelien-Antigen in Phosphat-gepufferter Saline i.p.
Tag 66:                Entnahme der Milz und Herstellung einer Einzelzellsuspension.

Die immunen Milzzellen wurden mit der Ag8-PAI Myelom-Zellinie nach Standardmethoden unter Verwendung von Polyethylenglycol fusioniert (J.H. Peters, H. Baumgarten, M. Schulze "Monoklonale Antikörper" Springer-Verlag, Heidelberg).

Die Fusionsprodukte wurden in 96 wells Gewebekulturplatten ausgesät. Nach 8 Tagen wurden die Zellkulturüberstände auf die Anwesenheit von B. burgdorferi spezifischen monoklonalen Antikörpern mit Hilfe eines Festphasen-ELISA, untersucht (J.H. Peters et al., s.o.).

Die Hybridoma-Zellen aus Antikörper-produzierenden Kulturen wurden nach der Grenzverdünnungsmethode kloniert. Die Kulturüberstände individueller Klone wurden anschließend erneut im Festphasen-ELISA sowie durch Western-Blot Analyse und durch Immunfluoreszenz-Untersuchungen charakterisiert. Der monoklonale Antikörper LA-2 der Subklasse IgG2b wird von einer monoklonalen Hybridoma-Linie produziert und sezerniert und reagiert im Western-Blot mit der 31kDa-Struktur, (Osp-A) von allen untersuchten B. burgdorferi Stämmen (u. a. die Isolate ZS7 und B31) bei Kontakt mit elektrophoretisch über ein SDS-Gel aufgetrennten und mittels Westernblot auf eine Membran transferierten B. burgdorferi Proteinen. Die monoklonalen Antikörper LA-26.1C(anti-ospA IgG1), LA 25.1 (anti-OspB (34 kDa Antigen); IgG2b) und LA 27.1 (anti-OspB (34 kDa Antigen), IgG1) wurden auf analoge Weise hergestellt und charakterisiert.

Infektion von Mäusen mit B. burgdorferi ZS7

C.B-17 Scid-Mäuse wurden mit 1x10$^8$ lebensfähigen B. burgdorferi ZS7-Organismen subkutan in die Schwanzwurzel infiziert.

Behandlung der Mäuse mit Antiseren

Die infizierten Scid-Mäuse wurden zweimal pro Woche mit verschiedenen Antiseren behandelt. Die eine Gruppe wurde mit NMS (normales Mausserum), eine zweite Gruppe mit IMS (immunes Mausserum) und eine dritte Gruppe mit dem monoklonalen Antikörper LA-2 (gegen das 31 kD Antigen von B. burgdorferi) behandelt. Die Dosis der verabreichten Antiseren betrug in der ersten Woche 100 $\mu$l bzw. 100 $\mu$g bei LA-2, in der zweiten Woche 200 $\mu$l bzw. 200 $\mu$g bei LA-2 und in der dritten Woche 300 $\mu$l bzw. 300 $\mu$g bei LA-2.

Tabelle 2 zeigt, daß unbehandelte oder mit NMS behandelte Scid-Mäuse nach 12 Tagen klinische und histopathologische Anzeichen von Arthrithis bzw. Carditis und Hepatitis entwickelten. Dagegen bewirkte die Verabreichung des monoklonalen Antikörpers LA-2 bei Scid-Mäusen eine deutliche Reduzierung der Symptome. Klinisch waren nur leichte Rötungen der Gelenke und histopathologisch nur marginale Veränderungen festzustellen. Mit IMS behandelte Mäuse zeigten keine klinischen Arthrithisbefunde.

Ein Nachweis des B. burgdorferi Erregers durch in vitro Kultivierung gelang nur bei Mäusen, die entweder unbehandelt oder mit NMS behandelt wurden. Bei den mit LA-2 oder IMS behandelten Mäusen konnte B. burgdorferi nicht nachgewiesen werden (Tabelle 2).

# TABELLE 2

**Behandlung von C.B-17 Scid-Mäusen mit B. burgdorferi und Antiseren**

| Mäusestamm C.B-17 scid | Behandlung mit Antiserum | Arthritis (nach 12 Tagen) | | Carditis/ Hepatitis | B. burgdorferi |
|---|---|---|---|---|---|
| | | klinisch | histopatho- logisch | histopatho- logisch | Nachweis (Anzucht) |
| n = 3 | – | + | + | + | + |
| n = 3 | NMS | + | + | + | + |
| n = 2 | IMS | – | – | – | – |
| n = 3 | LA-2 | –° | –°° | – | – |

° leichte Rötung des Gelenks

°° nur marginale Veränderung

EP 0 418 827 B1

**Beispiel 3**

Expressionsklonierung des 31kD Antigens (OspA) von B. burgdorferi ZS7

DNA-Präparation

Hochmolekulare DNA aus dem B. burgdorferi Stamm ZS7 wurde nach Kultivierung in modifiziertem Kelly's Medium gereinigt. Die Spirochäten wurden durch Zentrifugation bei 10.000 g pelletiert und dreimal in PBS-Puffer gewaschen. Das trockene Pellet wurde in 10 ml TE (10 mmol/l Tris, 1 mmol/l EDTA, pH 7,4) resuspendiert, mit Lysozym (5 mg/ml) 15 Minuten lang bei 30°C behandelt und die DNA durch Zugabe von 1 ml 20%igem SDS freigesetzt. Nach Zugabe von 1,5 ml NaCl (5 mol/l) wurde die Lösung mit einem gleichen Volumen an Phenol extrahiert, gefolgt von einer Extraktion mit Chloroform. Die DNA wurde dann durch Zugabe von 2 Volumina absolutem Ethanol und Inkubation bei -20°C über Nacht gefällt. Nach Zentrifugation wurde der Rückstand in 0,5 ml TE gelöst und mit DNAse freier RNAse A (20 $\mu$g/ml) 45 Minuten lang bei 55°C inkubiert, gefolgt von einer einstündigen Behandlung mit Proteinase K (0,1 $\mu$g/ml) bei 37°C. Die Lösung wurde auf 0,3 mol/l NaOAc eingestellt und mit Phenol-Chloroform wie oben beschrieben extrahiert. Nach Fällung mit Ethanol wurde die DNA wieder in TE aufgenommen.

Herstellung der Genbank

Hochmolekulare DNA wurde durch drei Sekunden lange Ultraschallbehandlung statistisch zerkleinert. T4-DNA-Polymerase (30 Minuten bei 37°C) und Klenow-Enzym (5 Minuten bei 20°C) wurden dazu verwendet, um die Enden der erzeugten DNA-Fragmente zu glätten. DNA mit glatten Enden wurde in die BamHI-Stelle eines Expressionsvektors pUEX1 unter Verwendung einer Adapter-Klonierungsstrategie ligiert (Bresan und Stanley (1987) Nucl. Acid. Res. S. 1056). Nach einem Größenselektionsschritt durch eine Molekularsieb-Chromatographie über Sephacryl S-1000 und Transformation von kompetenten Wirtszellen E. coli (MC 1061) wurde der Anteil an rekombinanten Plaque bildenden Einheiten (pfu) wie folgt bestimmt: zufällig ausgewählte Kolonien wurden gepickt und bis zur Sättigung in 2 ml Selektionsmedium (LB mit 25 $\mu$g/ml Ampicillin) angezüchtet. Die Plasmid-DNA wurde nach der üblichen alkalischen Lysis-Methode isoliert und anschließend mit BamHI geschnitten. Mehr als 50 % der analysierten Plasmide enthielten durchschnittlich $\geq$ 1,5 kb lange DNA-Insertionen.

Ausplattieren und Screening der B. burgdorferi ZS7-Genbank

Die Zellen wurden auf 24x24 cm Platten bei einer Dichte von 7.000 pfu pro Platte ausplattiert und über Nacht bei 30°C inkubiert. Nach dem Transfer der Kolonien auf Nitrocellulosefilter (NC) wurde die Expression von $\beta$-Galactosidase-Fusionsproteinen durch zweistündige Inkubation bei 42°C induziert. Die Filter wurden auf ein Whatman 3MM-Papier transferiert, das mit 5 % SDS behandelt worden war, und etwa 25 Minuten lang bei 95°C inkubiert. Dann wurden die Proteine elektrogeblottet unter Verwendung einer üblichen Apparatur zum halbtrockenen Westernblotting. Nach DNAse-Behandlung der NC-Filter wurden immunreaktive Klone durch ein Expressions-Screening unter Verwendung monoklonaler Antikörper identifiziert. Unspezifische Bindungsstellen auf den NC-Filtern wurden durch vierstündige Inkubation mit PBS, enthaltend 0,2 % (Gewicht pro Volumen) Gelatine und 3 mmol/l NaN$_3$ bei Raumtemperatur abgesättigt. Anschließend wurden die Filter mit Kulturüberständen des Anti-31 kD monoklonalen Antikörperklons LA-2 18 Stunden lang unter andauerndem Schütteln inkubiert. Nach gründlichem Waschen (PBS + 1 % (Volumen/Volumen) Triton X-100; PBS + 0,5 mol/l Natriumchlorid; PBS + 1 mol/l Natriumchlorid; jeder Schritt 10 Minuten) wurden die Filter mit der 1:10.000 Verdünnung einer Peroxidase-markierten F(ab)$_2$-Präparation von Kaninchen-Anti-Maus-IgG-Antikörpern 1,5 Stunden lang bei Raumtemperatur unter andauerndem Schütteln inkubiert. Die Filter wurden wieder, wie oben beschrieben, gewaschen und dann mit Diaminobenzidin als Peroxidasesubstrat inkubiert. Von 10$^4$ rekombinanten PFU's reagierten 20 Klone mit dem monoklonalen Antikörper LA-2.

Sequenzanalyse des 31 kD Antigens (OspA)

Die insertierte DNA eines rekombinanten E. coli Klons mit positiver Antikörperreaktion mit LA-2 wurde auf übliche Weise isoliert. Die DNA-Insertion dieses Klons enthielt das für das B. burgdorferi 31 kD Antigen kodierende ospA-Gen in voller Länge. Das Plasmid, das die Insertion enthält, wurde PZS-7/31-2 bezeichnet und wurde gemäß Budapester Vertrag bei DSM (unter der Nummer DSM 5528) hinterlegt.

Das von diesem immunpositiven Klon produzierte rekombinante Protein wurde als rZS7/31-2 bezeichnet. Die kodierende DNA-Sequenz des ospA-Gens wurde bestimmt. Sie ist zusammen mit der davon abgeleiteten Aminosäuresequenz des OspA Proteins in Abb. 1 dargestellt.

Aus Abb. 1 ist auch ersichtlich, daß das 31 kD Antigen von B. burgdorferi einem Protein mit 273 Aminosäuren entspricht.

## Präparation von Nicht-Fusionsproteinen

a) Der Klon, der das immunreaktive Protein rZS7/31-2 exprimiert, wurde über Nacht bei 30 °C in 10 ml LB mit Ampicillin gezüchtet. 1 ml der Kultur wurde in 100 ml Selektionsmedium eingebracht und bei 30 °C mit guter Belüftung bis zu einer Dichte von $8 \times 10^7$ Zellen pro ml (A600 = 0,2) gezüchtet. Die Expression des rekombinanten Proteins wurde durch einen Transfer der Wirtszellen auf 42 °C erreicht. Nach Abkühlen und Zentrifugation wurden die Zellen in STE-Puffer (10 mmol/l Tris, 100 mmol/l Natriumchlorid, 1 mmol/l EDTA, pH 8,0) gewaschen und der Rückstand in 0,6 ml Lysispuffer (25 % Sucrose, 50 mmol/l Tris, pH 8,0) resuspendiert. Nach Zugabe von 150 $\mu$l Lysozym (10 mg/ml) wurde die Mischung 15 Minuten lang auf Eis inkubiert, gefolgt von einer weiteren Inkubation (15 Minuten auf Eis) mit 18 $\mu$l DNAse 1 (10 mg/ml) in Gegenwart von 5 $\mu$l 1 mol/l Magnesiumchlorid. Schließlich wurden 250 $\mu$l 4x Detergensmix (1 % Triton X 100, 0,5 % Deoxycholat, 0,1 mol/l NaCl, 10 mmol/l Tris, pH 7,4) zugefügt und 5 Minuten lang auf Eis inkubiert. Nach Zentrifugation wurde der Rückstand zweimal mit Puffer A (50 mmol/l Tris, 50 mmol/l NaCl, 1 mmol/l EDTA, pH 8,0) gewaschen und in 9 Volumina Puffer A, zusätzlich 8 M Harnstoff enthaltend, resuspendiert und eine Stunde lang bei Raumtemperatur inkubiert. Die Probe wurde verdünnt mit 9 Teilen Puffer B (50 mmol/l $KH_2PO_4/K_2HPO_4$, 50 mol/l NaCl, 1 mmol/l EDTA, pH 10,7) und 30 Minuten lang bei Raumtemperatur gerührt, wobei der pH-Wert durch Zugabe von KOH bei 10,7 gehalten wurde. Nach Einstellung eines pH-Werts der Lösung auf 7,0 durch Zugabe von HCl wurde die Probe über Nacht gegen Puffer A dialysiert (bei 4 °C) und 10 Minuten lang bei 4 °C und 10.000 Umläufen pro Minute (Upm) in einem SS34-Rotor zentrifugiert. Der Überstand, der das rekombinante Protein enthält, wurde bei -20 °C aufbewahrt.

b) Da der Klon das immunreaktive Protein rZS7/31-2 auch in das Kulturmedium sezerniert, ist eine Reinigung (Affinitätchromatographie) direkt aus Kulturüberstand möglich.

## Präparation von rekombinanten OspA (Nicht-Fusions) Protein und Affinitäts-chromatografische Reinigung

Die rekombinanten Proteine wurden anschließend Affinitäts-chromatographisch gereinigt.

Zu diesem Zweck wurden gereinigte monoklonale Antikörper LA-2 an aktivierte Sepharose CL 4B kovalent gebunden. Der dialysierte Harnstoff-Extrakt mit dem rekombinanten Protein wurde an Maus IgG-Sepharose CL 4B adsorbiert und anschließend über die LA-2-Sepharose CL 4B Säule gegeben. Nach intensivem Waschen wurde das gebundene rekombinante Protein mit 0,1 mol/ Glycin/HCl - 0,1 mol/l NaCl, pH 2,5 eluiert. Der pH-Wert der gesammelten Fraktionen wurde durch sofortige Zugabe von 1/10 Vol. 0,5 mol/l $K_2HPO_4$ neutralisiert. Die proteinhaltigen Fraktionen wurden konzentriert und dialysiert. Mit Hilfe der SDS-Polyacrylamid Gel Elektrophorese wurde der Grad der Reinigung bestimmt.

## Immunologische Charakterisierung des rekombinanten Proteins rZS7/31-2

Das rekombinante Protein rZS7/31-2 wurde immunologisch untersucht. Zum Vergleich wurde das rekombinante Protein rB31/41-9 (B. burgdorferi 41 kD Oberflächenantigen) herangezogen.

Flachboden-Mikrotiterplatten wurden mit Harnstoffextrakten der rekombinanten Proteine rZS7/31-2 und rB31/41-9 bzw. einem Harnstoffextrakt des zur Genexpression verwendeten E. coli Stamms MC 1061 beschichtet. Unspezifische Bindungsstellen wurden mit 0,2 % Gelatine in phosphatgepufferter Kochsalzlösung blockiert. Vertiefungen der so vorbereiteten Mikrotiterplatten wurden mit den angegebenen monoklonalen Antikörpern LA-2 (anti-31 kD, Osp-A), LA-1 (anti-41 kD, Flagellin) bzw. ACHT-2 (anti-$\alpha_1$-Antichymotrypsin) in Ansatz gebracht.

Die gebundenen monoklonalen Antikörper wurden mit peroxidasemarkierten speziesspezifischen anti-Maus Immunglobulinen zur Reaktion gebracht. Gebundene peroxidasemarkierte Antikörper wurden unter Verwendung des Peroxidasesubstrats Orthophenylendiamin quantifiziert. Die Absorption bei 492 nm ($A_{492}$) wurde direkt in der Mikrotiterplatte mit Hilfe eines automatisierten Plattenphotometers bestimmt. Die Stärke der Absorption ist ein Maß für die Menge gebundener monoklonaler Antikörper.

Der monoklonale Antikörper LA-2 reagiert in spezifischer Weise mit rZS7/31-2 aber nicht mit MC 1061 bzw. rB31/41-9. Die Kontrollreaktion des monoklonalen Antikörpers LA-1 ist spezifisch für rB31/41-9. Der monoklonale Kontrollantikörper ACHT-2 (Negativkontrolle) zeigt auf keinem der Proteine eine signifikante Reaktion.

Abbildung 2 zeigt, daß das vom monoklonalen Antikörper LA-2 in spezifischer Weise erkannte antigene Epitop auf dem rekombinanten Protein rZS7/31-2 exprimiert ist, das aus dem Genom von B. burgdorferi ZS7 kloniert wurde.

**Beispiel 4**

Vergleich von für das 31 kD (OspA) bzw. 34 kD Antigen (OspB) spezifischen Antikörpern mit Antikörpern, die für das 41 kD Antigen (Flagellin) spezifisch sind

Die monoklonalen Antikörper LA-2 und LA-26.1 erkennen das 31 kD Antigen OspA und sind vom Isotyp IgG2b bzw. IgG1. Die monoklonalen Antikörper LA-25.1 und LA-27.1 erkennen das 34 kD Antigen OspB und sind vom Isotyp IgG2b bzw. IgG1. Die monoklonalen Antikörper LA-10 und LA-21 sind spezifisch für das Flagellen-assoziierte 41 kD periplasmatische Protein von B. burgdorferi und sind vom Isotyp IgG2a bzw. IgG1. Alle obengenannten Antikörper wurden nach dem in Beispiel 2 beschriebenen Verfahren erhalten. Es sollte in diesem Versuch festgestellt werden, ob auch monoklonale Antikörper gegen ein anderes B. burgdorferi Antigen in Scid-Mäusen eine Protektion vor den klinischen Symptomen der Lyme-Borreliose bewirken.

Das polyklonale Anti-B31-Immunserum (IMS) wurde aus C57BL/6-Mäusen 91 Tage nach einer subkutanen Inokkulation mit $1 \times 10^8$ B. burgdorferi B31-Organismen entnommen. Das polyklonale Anti-ZS7 IMS wurde aus C57BL/6-Mäusen 68 Tage nach einer subkutanen Inokulation mit $1 \times 10^8$ B. burgdorferi ZS7 entnommen. Beide Sera enthielten 60 $\mu$g/ml spezifische Antikörper, wie in einem ELISA-System bestimmt wurde (Schaible et al., J. Exp. Med. 170 (1989), 1427-1432). Das Normal-Maus-Serum (NMS) wurde aus nicht infizierten C57BL/6-Mäusen entnommen.

Zum Zeitpunkt der Inokulation und darauf folgend in 4-Tages-Intervallen wurden die angegebenen Antikörper, das IMS, das NMS oder PBS-Puffer passiv intraperitoneal in Scid-Mäuse gemäß folgendem Protokoll transferiert:

Tag 0 und Tag 3: 100 $\mu$l,

Tag 7 und Tag 10: 200 $\mu$l,

Tag 13 und Tag 17: 300 $\mu$l.

Scid-Mäuse, die entweder mit Anti-ZS7IMS, Anti-B31IMS oder mit dem monoklonalen Antikörper LA-2 behandelt wurden, zeigten keine sichtbaren klinischen Symptome von Arthritis, d.h. es trat keine Rötung und Schwellung von Tibioltarsalgelenken während der 21 Beobachtungstage auf. Ebenso waren keine Symptome von Carditis und Hepatitis festzustellen. Histopathologische Untersuchungen ergaben keine Veränderungen in den Gelenken, dem Herzen und der Leber von Scid-Mäusen, die entweder mit Anti-ZS7-IMS, Anti-B31-IMS oder mit dem monoklonalen Antikörper LA-2 behandelt wurden.

Auch der andere OspA-spezifische monoklonale Antikörper LA-26.1 vom Isotyp IgG1 sowie die OspB spezifischen Antikörper LA-25.1 und LA-27.1 waren in der Lage, die klinischen Symptome von Arthritis, Carditis und Hepatitis zu mildern. Hier zeigten sich leichte pathologische Veränderungen in den untersuchten Organen.

Im Gegensatz dazu zeigten Scid-Mäuse, die entweder PBS-Puffer, NMS oder monoklonale Antikörper gegen Flagellin (LA-10 oder LA-21) verabreicht bekommen hatten, klinische Zeichen von Arthritis, die für unbehandelte Scid-Mäuse typischen, pathologischen Veränderungen (Tabelle 3). Die Schwere der Symptome in den letztgenannten Tieren war mit zunehmender Zeitdauer nach der Inokkulation ansteigend und schwächte sich während der gesamten Beobachtungsperiode nicht ab. Aus Scid-Mäusen, die zuvor entweder mit Anti-ZS7IMS oder mit dem Antikörper LA-2 behandelt wurden, konnten keine Spirochäten isoliert werden. Im Gegensatz dazu war der Nachweis von Spirochäten durch Immunfluoreszenz und durch Kultivierung aus dem Blut von Scid-Mäusen möglich, die mit PBS-Puffer, NMS oder den monoklonalen Antikörpern LA-25.1, LA-26.1, LA-27.1, LA-10 oder LA-21 behandelt worden waren.

T a b e l l e   3

| Mäusestamm C.B.-17 Scid (Anzahl der Mäuse) | Behandlung mit | IgG Subtyp | klinische Arthritis | Histopathologie Periarthritis/ Arthritis | Carditis | Nachweis von B. burgdorferi (Kultivierung und Immunfluoreszenz) |
|---|---|---|---|---|---|---|
| n=8 | PBS (negative Kontrolle) | | + | + | + | + |
| n=3 | NMS (negative Kontrolle) | | + | + | + | + |
| n=3 | anti-B31 IMS | | − | − | − * | + o |
| n=2 | anti-ZS7 IMS | | − | − | − * | − |
| n=6 | LA-2 (OspA) | 2b | − | − * | − * | − |
| n=3 | LA-10 (Flagellin) | 2a | + | + | + | + o |
| n=3 | LA-21 (Flagellin) | 1 | + | + | +/− | + |
| n=3 | LA-26.1 (OspA) | 1 | ± | ± | ± | + o |
| n=3 | LA-25.1 (OspB) | 2b | ± | ± | ± | + |
| n=3 | LA-27.1 (OspB) | 1 | ± | ± | ± | + o |

Der Grad der histopathologischen Veränderung ist wie folgt angegeben: − keine, − * subklinisch, +/− mäßig, + schwere.

o Ein B. burgdorferi Nachweis durch Immunfluoreszenz war nicht in allen untersuchten Individuen möglich.

EP 0 418 827 B1

**Patentansprüche**

**Patentansprüche für folgende Vertragsstaaten : AT, BE, CH, DE, DK, FR, GB, IT, LI, LU, NL, SE**

1. Impfstoff gegen die Lyme-Krankheit,
   **dadurch gekennzeichnet,**
   daß er einen oder mehrere monoklonale Antikörper enthält, die spezifisch für das 31 kD Antigen (OspA) oder das 34 kD Antigen (OspB) von B. burgdorferi sind.

2. Impfstoff nach Anspruch 1, **dadurch gekennzeichnet,** daß der Antikörper spezifisch für das 31 kD oder das 34 kD Antigen von B. burgdorferi der Stämme ZS7 (DSM 5527) oder/und B31 (ATCC 35210) ist.

3. Impfstoff nach Anspruch 1 oder 2,
   **dadurch gekennzeichnet,**
   daß er einen monoklonalen Antikörper der Klasse IgG, vorzugsweise der Subklasse IgG2b oder IgG1, als Wirkstoff enthält.

4. Impfstoff nach einem der Ansprüche 1 bis 3,
   **dadurch gekennzeichnet,**
   daß er in immundefizienten Versuchstieren, die mit lebensfähigen pathogenen B. burgdorferi Organismen infiziert wurden, die Ausbildung von Arthritis, Carditis und Hepatitis verhindert.

5. Impfstoff nach einem der Ansprüche 1 bis 4,
   **dadurch gekennzeichnet,**
   daß er in immundefizienten Scid-Mäusen, die mit lebensfähigen B. burgdorferi Organismen, Stamm ZS7, infiziert wurden, die Ausbildung von Arthritis, Carditis und Hepatitis weitgehend verhindert.

6. Impfstoff nach einem der Ansprüche 1 bis 5,
   **dadurch gekennzeichnet,**
   daß er den monoklonalen Antikörper LA2 gegen OspA, sekretiert von der Hybridoma-Zellinie, ECACC 89 09 1302, enthält.

7. Impfstoff nach einem der Ansprüche 1 bis 5,
   **dadurch gekennzeichnet,**
   daß er den monoklonalen Antikörper LA 26.1 gegen OspA, sekretiert von der Hybridoma-Zellinie, ECACC 90050406, enthält.

8. Impfstoff nach einem der Ansprüche 1 bis 5,
   **dadurch gekennzeichnet,**
   daß er den monoklonalen Antikörper LA 25.1 gegen OspB, sekretiert von der Hybridoma-Zellinie, ECACC 90050405, enthält.

9. Impfstoff nach einem der Ansprüche 1 bis 5,
   **dadurch gekennzeichnet,**
   daß er den monoklonalen Antikörper LA 27.1 gegen OspB, sekretiert von der Hybridoma-Zellinie, ECACC 90050407, enthält.

**Patentansprüche für folgende Vertragsstaaten : ES, GR**

1. Verfahren zur Herstellung eines Impfstoffs gegen die Lyme-Krankheit,
   **dadurch gekennzeichnet,**
   daß man einen oder mehrere monoklonale Antikörper verwendet, die spezifisch für das 31 kD Antigen (OspA) oder das 34 kD Antigen (OspB) von B. burgdorferi sind.

2. Verfahren nach Anspruch 1,
   **dadurch gekennzeichnet,**
   daß der Antikörper spezifisch für das 31 kD oder das 34 kD Antigen von B. burgdorferi der Stämme ZS7 (DSM 5527) oder/und B31 (ATCC 35210) ist.

14

3. Verfahren nach Anspruch 1 und 2,
   **dadurch gekennzeichnet,**
   daß der Impfstoff einen monoklonalen Antikörper der Klasse IgG, vorzugsweise der Subklasse IgG2b oder IgG1, als Wirkstoff enthält.

4. Verfahren nach einem der Ansprüche 1 bis 3,
   **dadurch gekennzeichnet,** daß der Impfstoff in immundefizienten Versuchstieren, die mit lebensfähigen pathogenen B. burgdorferi Organismen infiziert wurden, die Ausbildung von Arthritis, Carditis und Hepatitis verhindert.

5. Verfahren nach einem der Ansprüche 1 bis 4,
   **dadurch gekennzeichnet,**
   daß der Impfstoff in immundefizienten Scid-Mäusen, die mit lebensfähigen B. burgdorferi Organismen, Stamm ZS7, infiziert wurden, die Ausbildung von Arthritis, Carditis und Hepatitis weitgehend verhindert.

6. Verfahren nach einem der Ansprüche 1 bis 5,
   **dadurch gekennzeichnet,**
   daß der Impfstoff den monoklonalen Anikörper LA2 gegen OspA, sekretiert von der Hybridoma-Zellinie, ECACC 89 09 1302, enthält.

7. Verfahren nach einem der Ansprüche 1 bis 5,
   **dadurch gekennzeichnet,**
   daß der Impfstoff den monoklonalen Antikörper LA 26.1 gegen OspA, sekretiert von der Hybridoma-Zellinie, ECACC 9005046, enthält.

8. Verfahren nach einem der Ansprüche 1 bis 5,
   **dadurch gekennzeichnet,**
   daß der Impfstoff den monoklonalen Antikörper LA 25.1 gegen OspB, sekretiert von der Hybridoma-Zellinie, ECACC 90050405, enthält.

9. Verfahren nach einem der Ansprüche 1 bis 5,
   **dadurch gekennzeichnet,**
   daß der Impfstoff den monoklonalen Antikörper LA 27.1 gegen OspB, sekretiert von der Hybridoma-Zellinie, ECACC 90050407, enthält.

**Claims**
**Claims for the following Contracting States : AT, BE, CH, DE, DK, FR, GB, IT, LI, LU, NL, SE**

1. Vaccine against lyme disease, characterised in that it contains one or more monoclonal antibodies which are specific for the 31 kD antigen (OspA) or the 34 kD antigen (OspB) of B. burgdorferi.

2. Vaccine according to claim 1, characterised in that the antibody is specific for the 31 kD or the 34 kD antigen of B. burgdorferi of the strains ZS7 (DSM 5525) and/or B31 (ATCC 35210).

3. Vaccine according to claim 1 or 2, characterised in that it contains as active material a monoclonal antibody of the class IgG, preferably of the subclass IgG2b or IgG1.

4. Vaccine according to one of claims 1 to 3, characterised in that it prevents the, formation of arthritis, carditis and hepatitis in immune-deficient experimental animals which have been infected with viable, pathogenic B. burgdorferi organisms.

5. Vaccine according to one of claims 1 to 4, characterised in that it substantially prevents the formation of arthritis, carditis and hepatitis in immune-deficient scid mice which have been infected with viable B. burgdorferi organisms, strain ZS7.

6. Vaccine according to one of claims 1 to 5, characterised in that it contains the monoclonal antibody LA-2 against OspA, secreted by the hybridoma cell line ECACC 89091302.

7. Vaccine according to one of claims 1 to 5, characterised in that it contains the monoclonal antibody LA-26.1 against OspA secreted by the hybridoma cell line ECACC 90050406.

8. Vaccine according to one of claims 1 to 5, characterised in that it contains the monoclonal antibody LA-25.1 against OspB secreted by the hybridoma cell line ECACC 90050405.

9. Vaccine according to one of claims 1 to 5, characterised in that it contains the monoclonal antibody LA-27.1 against OspB secreted by the hybridoma cell line ECACC 90050407.

**Claims for the following Contracting States : ES, GR**

1. Process for the preparation of a vaccine against lyme disease, characterised in that one uses one or more monoclonal antibodies which are specific for the 31 kD antigen (OspA) or the 34 kD antigen (OspB) of B. burgdorferi.

2. Process according to claim 1, characterised in that the antibody is specific for the 31 kD or the 34 kD antigen of B. burgdorferi of the strain ZS7 (DSM 5527) and/or B31 (ATCC 35210).

3. Process according to claims 1 and 2, characterised in that the vaccine contains a monoclonal antibody of the class IgG, preferably of the subclass IgG2b or IgG1 as active material.

4. Process according to one of claims 1 to 3, characterised in that the vaccine prevents the formation of arthritis, carditis and hepatitis in immune-deficient experimental animals which have been infected with viable pathogenic B. burgdorferi organisms.

5. Process according to one of claims 1 to 4, characterised in that the vaccine prevents the formation of arthritis, carditis and hepatitis in immune-deficient scid mice which have been infected with viable B. burgdorferi organisms, strain ZS7.

6. Process according to one of claims 1 to 5, characterised in that the vaccine contains the monoclonal antibody LA2 against OspA secreted by the hybridoma cell line ECACC 89091302.

7. Process according to one of claims 1 to 5, characterised in that the vaccine contains the monoclonal antibody LA26.1 against OspA secreted by the hybridoma cell line ECACC 90050406.

8. Process according to one of claims 1 to 5, characterised in that the vaccine contains the monoclonal antibody LA25.1 against OspB secreted by the hybridoma cell line ECACC 90050405.

9. Process according to one of claims 1 to 5, characterised in that the vaccine contains the monoclonal antibody LA27.1 against OspB secreted by the hybridoma cell line ECACC 90050407.

**Revendications**
**Revendications pour les Etats contractants suivants : AT, BE, CH, DE, DK, FR, GB, IT, LI, LU, NL, SE**

1. Vaccin contre la maladie de Lyme, caractérisé en ce qu'il contient un ou plusieurs anticorps monoclonaux qui sont spécifiques de l'antigène 31 kD (OspA) ou de l'antigène 34 kD (OspB) de B. burgdorferi.

2. Vaccin selon la revendication 1, caractérisé en ce que l'anticorps est spécifique de l'antigène 31 kD ou 34 kD de B. burgdorferi des souches ZS7 (DSM 5527) ou/et B31 (ATCC 35210).

3. Vaccin selon la revendication 1 ou 2, caractérisé en ce qu'il contient en tant que principe actif un anticorps monoclonal de la classe IgG, de préférence de la sous-classe IgG2b ou IgG1.

4. Vaccin selon l'une des revendications 1 à 3, caractérisé en ce qu'il empêche le développement de l'arthrite, de la cardite et de l'hépatite chez des animaux d'essai immunodéficients qui ont été infectés par des organismes B. burgdorferi pathogènes viables.

5. Procéde selon l'une des revendications 1 à 4, caractérisé en ce qu'il empêche dans une large mesure le développement de l'arthrite, de la cardite et de l'hépatite chez des souris Scid immunodéficientes qui ont été infectées par des organismes B. burgdorferi viables, souche ZS7.

6. Vaccin selon l'une des revendications 1 à 5, caractérisé en ce qu'il contient l'anticorps monoclonal LA2 contre OspA, sécrété par la lignée de cellules d'hybridome ECACC 89 09 1302.

7. Vaccin selon l'une des revendications 1 à 5, caractérisé en ce qu'il contient l'anticorps monoclonal LA 26.1 contre OspA, sécrété par la lignée de cellules d'hybridome ECACC 90050406.

8. Vaccin selon l'une des revendications 1 à 5, caractérisé en ce qu'il contient l'anticorps monoclonal LA 25.1 contre OspB, sécrété par la lignée de cellules d'hybridome ECACC 90050405.

9. Vaccin selon l'une des revendications 1 à 5, caractérisé en ce qu'il contient l'anticorps monoclonal LA 27.1 contre OspB, sécrété par la lignée de cellules d'hybridome ECACC 90050407.

**Revendications pour les Etats contractants suivants : ES, GR**

1. Procédé de préparation d'un vaccin contre la maladie de Lyme, caractérisé en ce qu'on utilise un ou plusieurs anticorps monoclonaux qui sont spécifiques de l'antigène 31 kD (OspA) ou de l'antigène 34 kD (OspB) de B. burgdorferi.

2. Procédé selon la revendication 1, caractérisé en ce que l'anticorps est spécifique de l'antigène 31 kD ou 34 kD de B. burgdorferi des souches ZS7 (DSM 5527) ou/et B31 (ATCC 35210).

3. Procédé selon les revendications 1 et 2, caractérisé en ce que le vaccin contient en tant que principe actif un anticorps monoclonal de la classe IgG, de préférence de la sous-classe IgG2b ou IgG1.

4. Procédé selon l'une des revendications 1 à 3, caractérisé en ce que le vaccin empêche le développement de l'arthrite, de la cardite et de l'hépatite chez des animaux d'essai immunodéficients qui ont été infectés par des organismes B. burgdorferi pathogènes viables.

5. Procédé selon l'une des revendications 1 à 4, caractérisé en ce que le vaccin empêche dans une large mesure le développement de l'arthrite, de la cardite et de l'hépatite chez des souris Scid immunodéficientes que ont été infectées par des organismes B. burgdorferi viables, souche ZS7.

6. Procédé selon l'une des revendications 1 à 5, caractérisé en ce que le vaccin contient l'anticorps monoclonal LA2 contre OspA, sécrété par la lignée de cellules d'hybridome ECACC 89 09 1302.

7. Procédé selon l'une des revendications 1 à 5, caractérisé en ce que le vaccin contient l'anticorps monoclonal LA 26.1 contre OspA, sécrété par la lignée de cellules d'hybridome ECACC 9005046.

8. Procédé selon l'une des revendications 1 à 5, caractérisé en ce que le vaccin contient l'anticorps monoclonal LA 25.1 contre OspB, sécrété par la lignée de cellules d'hybridome ECACC 90050405.

9. Procédé selon l'une des revendications 1 à 5, caractérisé en ce que le vaccin contient l'anticorps monoclonal LA 27.1 contre OspB, sécrété par la lignée de cellules d'hybridome ECACC 90050407.

# Fig.1

DNA-Sequenz mit abgeleiteter Aminosäuresequenz
des 31kDa Antigens (OspA) aus B.burgdorferi ZS7.

```
atgaaaaaatatttattgggaataggtctaatattagccttaatagcatgtaagcaaaat
 M  K  K  Y  L  L  G  I  G  L  I  L  A  L  I  A  C  K  Q  N

gttagcagccttgacgagaaaaacagcgtttcagtagatttgcctggtgaaatgaacgtt
 V  S  S  L  D  E  K  N  S  V  S  V  D  L  P  G  E  M  N  V

cttgtaagcaaagaaaaaaacaaagacggcaagtacgatctaattgcaacagtagacaag
 L  V  S  K  E  K  N  K  D  G  K  Y  D  L  I  A  T  V  D  K

cttgagcttaaaggaacttctgataaaaacaatggatctggagtacttgaaggcgtaaaa
 L  E  L  K  G  T  S  D  K  N  N  G  S  G  V  L  E  G  V  K

gctgacaaaagtaaagtaaaattaacaatttctgacgatctaggtcaaaccacacttgaa
 A  D  K  S  K  V  K  L  T  I  S  D  D  L  G  Q  T  T  L  E

gttttcaaagaagatggcaaaacactagtatcaaaaaaagtaacttccaaagacaagtca
 V  F  K  E  D  G  K  T  L  V  S  K  K  V  T  S  K  D  K  S

tcaacagaagaaaaattcaatgaaaaaggtgaagtatctgaaaaaataataacaagagca
 S  T  E  E  K  F  N  E  K  G  E  V  S  E  K  I  I  T  R  A

gacggaaccagacttgaatacacagaaattaaaagcgatggatctggaaaagctaaagag
 D  G  T  R  L  E  Y  T  E  I  K  S  D  G  S  G  K  A  E

gttttaaaaagctatgttcttgaaggaactttaactgctgaaaaaacaacattggtggtt
 V  L  K  S  Y  V  L  E  G  T  L  T  A  E  K  T  T  L  V  V

aaagaaggaactgttactttaagcaaaaatatttcaaaatctggggaagtttcagttgaa
 K  E  G  T  V  T  L  S  K  N  I  S  K  S  G  E  V  S  V  E

cttaatgacactgacagtagtgctgctactaaaaaaactgcagcttggaattcaggcact
 L  N  D  T  D  S  S  A  A  T  K  K  T  A  A  W  N  S  G  T

tcaactttaacaattactgtaaacagtaaaaaaactaaagaccttgtgtttacaaaagaa
 S  T  L  T  I  T  V  N  S  K  K  T  K  D  L  V  F  T  K  E

aacacaattacagtacaacaatacgactcaaatggcaccaaattagagggggtcagcagtt
 N  T  I  T  V  Q  Q  Y  D  S  N  G  T  K  L  E  G  S  A  V

gaaattacaaaacttgatgaaattaaaaacgctttaaaataa
 E  I  T  K  L  D  E  I  K  N  A  L  K  *
```

# Fig.2

Immunologische Charakterisierung des
rekombinanten Proteins rZS7/31-2